# EUROPÄISCHE PATENTSCHRIFT

(11) **EP 0 955 021 B1**
(45) Veröffentlichungstag und Bekanntmachung des Hinweises auf die Patenterteilung: **19.09.2001**
(21) Anmeldenummer: 98104731.9
(22) Anmeldetag: 13.03.1998
(51) Int. Cl.: A61F 2/44

(54) **Satz von Bandscheiben-Endoprothesen**
Set of intervertebral disc endoprostheses
Jeu d'endoprothèses pour disques intervertébraux

(43) Veröffentlichungstag der Anmeldung: 10.11.1999
(73) Patentinhaber: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(56) Entgegenhaltungen:
- WO-A-93/10725
- US-A- 5 676 701
- US-A- 5 683 465

## Beschreibung

Es sind Bandscheiben-Endoprothesen bekannt (DE-C-35 29 761, US-A-5,676,701), die zwei Abschlußplatten aufweisen, die mit je einem Wirbelkörper zu verbinden sind. Die Abschlußplatten wirken unmittelbar oder vermittelst eines Gelenkkerns über komplementäre, meist sphärische Gelenkflächen zusammen, die ihnen im Verhältnis zueinander Dreh- und Neigebewegungen gestatten. Da die zu ersetzenden Bandscheiben unterschiedliche Größe haben, werden diese Bandscheiben-Endoprothesen in einem Satz unterschiedlicher Größenstufen angeboten, die in ihrer Gesamtheit allen normalerweise vorkommenden Größenanforderungen gerecht werden.

Unterschiedliche Größe von Wirbelkörpern kommt nicht nur bei unterschiedlichen Individuen vor. Vielmehr können auch in einer Wirbelsäule Wirbelkörper beträchtlich unterschiedlicher Abmessung benachbart sein. In diesen Fällen muß man zum Ersatz der zwischenliegenden Bandscheibe sich zwischen zwei Größenstufen entscheiden, von denen möglicherweise jede an der einen bzw. anderen Seite nicht recht paßt. Es wäre aber zu aufwendig, Zwischengrößen einzuführen.

Die Erfindung löst dieses Problem dadurch, daß ein Zwischenstufenelement vorgesehen ist, das mindestens auf einer Seite eine Gelenkfläche bildet und dessen Gelenkflächenmaße einer anderen Größenstufe angehören als seine Anschlußflächenmaße auf der der Gelenkfläche gegenüberliegenden Seite. Dieses Zwischenstufenelement gestattet es, Abschlußplatten miteinander in einer Bandscheiben-Endoprothese zu kombinieren, die unterschiedlichen Größenstufen angehören. So kann man auf beiden Seiten der Prothese unterschiedlichen Größenerfordernissen gerecht werden.

Die praktische Ausführung dieses Erfindungsgedankens kann in unterschiedlicher Weise geschehen. In einer ersten Ausführungsform wird das Zwischenstufenelement von einer Abschlußplatte gebildet. Die Außenmaße dieser Abschlußplatte gehören einer ersten Größenstufe an, während ihre Gleitflächenmaße einer zweiten Größenstufe angehören. So ist sie mit einer zweiten Abschlußplatte kombinierbar, die der zweiten Größenstufe angehört.

In einer anderen Ausführungsform wird das Zwischenstufenelement von einem Lagerelement gebildet, das auf der einen Seite eine Gelenkfläche aufweist, die einer ersten Größenstufe angehört. Auf der anderen Seite hat es Anschlußflächen, die zu komplementären Anschlußflächen von Abschlußplatten passen, die einer zweiten Größenklasse angehören. Diese Ausführungsform ist dann vorteilhaft, wenn Gelenkflächen aus unterschiedlichem Material gepaart werden sollen, von denen eine aus einem gleitgünstigem Kunststoff, z.B. HDPE, besteht. In diesem Fall wird das Lagerelement aus dem leichter zu bearbeitenden Werkstoff hergestellt. Daher kann die Zwischengröße zu geringen Herstellungs- und Lagerkosten bereitgehalten werden.

Eine Variante dieser Ausführungsform gilt für diejenigen Bandscheiben-Endoprothesen, die zwischen den Abschlußplatten einen Gelenkkern aufweisen, der mit jeder der beiden Abschlußplatten ein Gelenkflächenpaar bildet. Als Zwischenstufenelement wird in diesem Fall der Gelenkkern ausgewählt und mit unterschiedlichen Größenstufen angehörenden Gelenkflächenmaßen auf beiden Seiten versehen, so daß er mit Abschlußplatten unterschiedlicher Größenstufen kombiniert werden kann.

Der Grundgedanke der Erfindung besteht in der Kombinierbarkeit von Abschlußplatten, die unterschiedlichen Größenstufen angehören. Die Erfindung umfaßt daher auch eine solche Ausführung, bei der die Gelenkflächenmaße sämtlicher Abschlußplatten unterschiedlicher Größenstufen übereinstimmen und daher die Abschlußplatten beliebig kombinierbar sind.

Die Erfindung wird im folgenden näher unter Bezugnahme auf die Zeichnung erläutert, die vorteilhafte Ausführungsbeispiele veranschaulicht. Darin zeigen:
- Fig. 1 bis 3: eine erste Ausführungsform,
- Fig. 4 bis 6: eine zweite Ausführungsform und
- Fig. 7 bis 9: eine dritte Ausführungsform der Erfindung.

Sämtliche Bandscheiben-Endoprothesen weisen eine obere Abschlußplatte 1 und eine untere Abschlußplatte 2 auf, deren Anschlußflächen 3 mit den Wirbelkörpern zu verbinden sind, die die zu ersetzende Bandscheibe einschließen. Diese Anschlußflächen 3 können in geeigneter Weise im Hinblick auf ihre Verbindungseigenschaften zum Knochen ausgebildet werden, Beispielsweise können Dornen 4 und nicht dargestellte Schraubenlöcher vorgesehen sein. Die Abschlußplatten bestehen aus einem widerstandsfähigen, zur Übertragung der Last geeigneten Werkstoff, insbesondere einem der für Endoprothesen bewährten Metalle bzw. Legierungen.

Die Abschlußplatten bilden miteinander ein Gelenk, dessen Ausführung weiter unten erläutert wird.

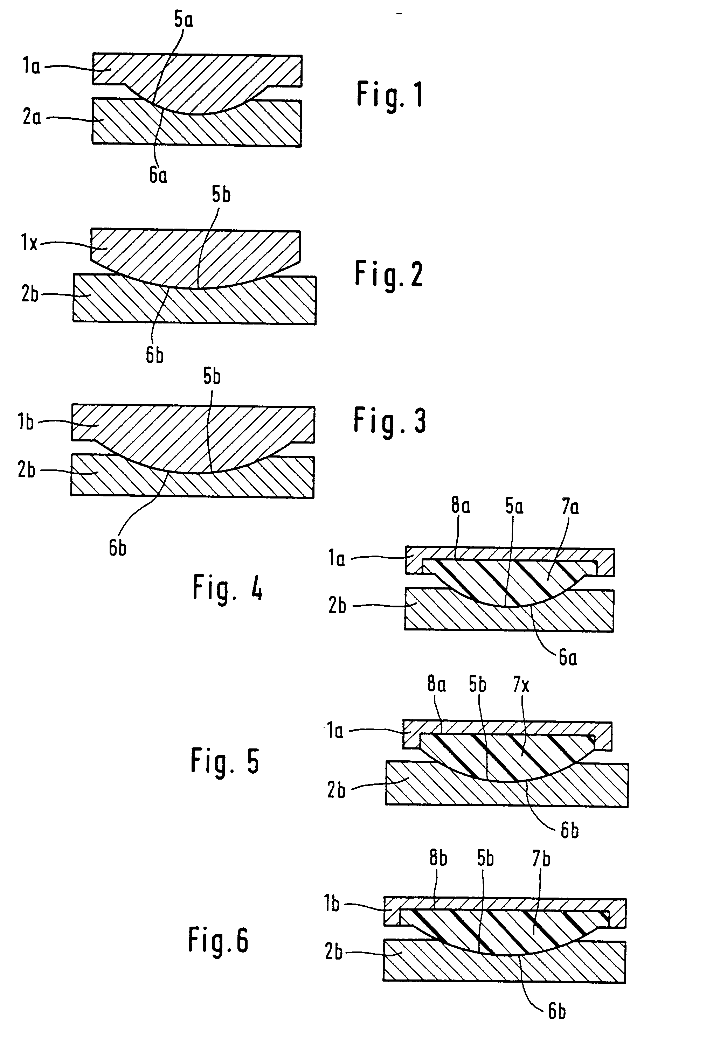

Die erste und die dritte Darstellung jedes Beispiels (Fig. 1/3, Fig. 4/6, Fig. 7/9) zeigen jeweils Prothesen, die zueinander geometrisch ähnlich sind; sie gehören jeweils unterschiedlichen, benachbarten Größenstufen a bzw. b an. Diese verschiedenen Größenstufen bilden - gegebenenfalls zusammen mit weiteren, nicht dargestellten Größenstufen - einen Satz verschieden großer Bandscheiben-Endoprothesen. Jeweils in der mittleren Fig. (Fig. 2,5,8) ist eine Prothese dargestellt, die erfindungsgemäß als Zwischengröße ausgebildet ist.

Die Abschlußplatten 1 und 2 schließen miteinander ein von Gelenkflächen 5,6 gebildetes Gelenk ein. Für die Erfindung kommt es nicht darauf an, wie dieses Gelenk ausgeführt ist. Die vorliegende Beschreibung bezieht sich einfachheitshalber auf solche Gelenke, die von etwa sphärischen, komplementären Gelenkflächenpaaren gebildet werden. Es kommt im vorliegenden Zusammenhang auch nicht darauf an, ob die Abschlußplatte mit der konvexen/konkaven Gelenkfläche oben bzw. unten angeordnet ist.

Im Ausführungsbeispiel gemäß Fig. 1 bis 3 sind die Gelenkflächen 5,6 unmittelbar an den Abschlußplatten 1,2 vorgesehen. Zur Bildung einer Zwischenstufe ist gemäß Fig. 2 eine Abschlußplatte, die der Größenstufe b angehört, mit einer Abschlußplatte 1x gepaart, deren Außenmaße denjenigen der Abschlußplatte 1a gleichen. In dieser Beziehung gehört sie somit der Größenstufe a an. Ihre Gelenkfläche 5 hat jedoch die Maße der zur Größenstufe b gehörenden Gelenkflächen 5b. Die Abschlußplatte 1x bildet auf diese Weise ein Zwischenelement, das mit einer in der Größenstufe b bereits vorhandenen Abschlußplatte eine Zwischengröße ergibt.

Die Ausführungsform gemäß Fig. 4 bis 6 gleicht derjenigen gemäß Fig. 1 bis 3 mit dem Unterschied, daß die Abschlußplatten 1 nicht unmittelbar die zugehörige Gelenkfläche 5 bilden, sondern vermittelst eines Lagerelements 7, das über komplementäre Verbindungsflächen 8 mit der zugehörigen Abschlußplatte 1a verbunden ist. Die Maße dieser Verbindungsflächen sind in den verschiedenen Größenstufen unterschiedlich. Eine Zwischenstufe zwischen den Größenstufen a (Fig. 4) und b (Fig. 6) wird gemäß Fig. 5 dadurch gebildet, daß ein Lagerelement 7x gebildet wird, das einerseits die zur Größenstufe a gehörigen Maße der Verbindungsfläche 8a aufweist. Andererseits gehören die Maße seiner Gelenkfläche 5b zur Größenstufe b. Auf diese Weise lassen sich die Abschlußplatten 1a und 2b miteinander zur Bildung einer Zwischenstufe paaren.

Im gezeigten Beispiel bildet das Lagerelement 7 die konvexe Gelenkfläche 5. Statt dessen ist es auch möglich, ein Lagerelement mit konkaver Lagerfläche vorzusehen. Das Lagerelement 7 besteht vorzugsweise aus leicht bearbeitbarem Werkstoff wie HDPE.

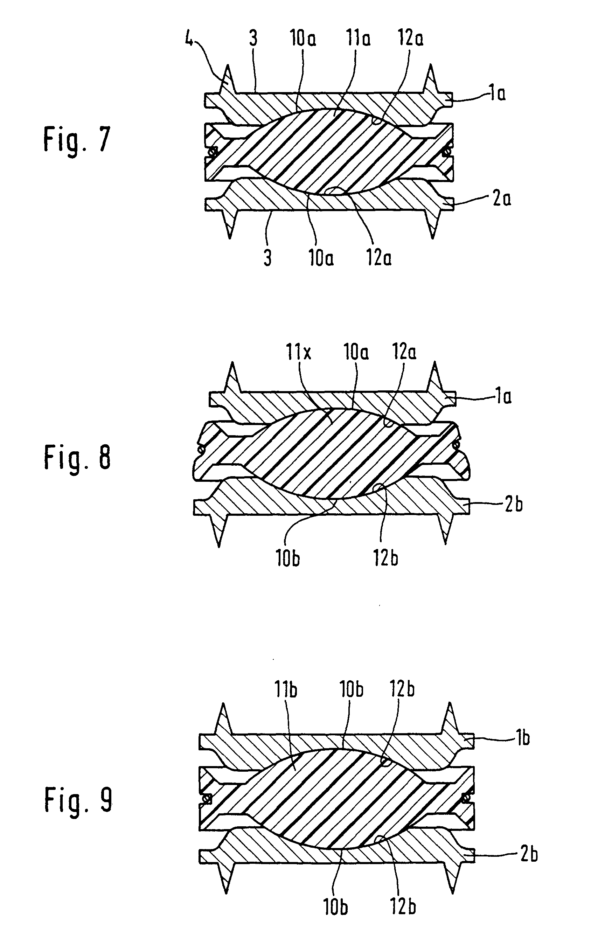

Die im Ausführungsbeispiel gemäß Fig. 7 bis 9 gezeigten Prothesen gehören dem Typ an, bei dem die beiden Abschlußplatten 1,2 jeweils unmittelbar mit einer Gelenkfläche 10 versehen sind, die vorzugsweise konkav ist. Sie bestehen aus festem Werkstoff wie Metall und schließen einen Gelenkkern 11 ein, der komplementäre Gleitflächen 12 bildet. Er besteht zweckmäßigerweise aus leicht bearbeitbarem, gleitgünstigem Kunststoff wie HDPE. Die Abschlußplatten 1,2 und die Gelenkkerne 11 unterscheiden sich in ihrer Größe bei den unterschiedlichen Größenstufen.

Eine Zwischenstufe wird gemäß Fig. 8 dadurch gebildet, daß der Gelenkkern 11x auf der einen Seite eine Gelenkfläche der Größenstufe 12a und auf der anderen Seite eine Gelenkfläche der Größenstufe 12b aufweist, so daß er einerseits mit einer Abschlußplatte 1a und andererseits mit einer Abschlußplatte 2b kombiniert werden kann.

## Patentansprüche

1. Satz von Bandscheiben-Endoprothesen bestehend aus mindestens zwei Größenstufen (a,b) von Bandscheiben-Endoprothesen, die zwei mit je einem Wirbelkörper zu verbindende, über Gelenkflächen zusammenwirkende Abschlußplatten (1,2) umfassen, deren Außen- und Gelenkflächenmaße sich von Größenstufe zu Größenstufe unterscheiden, **dadurch gekennzeichnet, daß** ein eine Gelenkfläche (5) bildendes Zwischenstufenelement (1x,7x,11x) vorgesehen ist, dessen Gelenkflächenmaße einer anderen Größenstufe angehören als seine Anschlußflächenmaße auf seiner der Gelenkfläche gegenüberliegenden Seite.

2. Satz von Bandscheiben-Endoprothesen nach Anspruch 1, **dadurch gekennzeichnet, daß** das Zwischenstufenelement (1x) eine Abschlußplatte ist.

3. Satz von Bandscheiben-Endoprothesen nach Anspruch 1, **dadurch gekennzeichnet, daß** das Zwischenstufenelement (7x) ein über Verbindungsflächen (8) von einer Abschlußplatte (1,2) gehaltenes Lagerelement (7) ist, dessen Anschlußflächenmaße einer anderen Größenstufe als seine Gelenkflächenmaße angehören.

4. Satz von Bandscheiben-Endoprothesen nach Anspruch 1, **dadurch gekennzeichnet, daß** die Bandscheiben-Endoprothesen zwischen jeder Abschlußplatte (1,2) und einem Gelenkkern (11) je ein Gelenkflächenpaar (10,12) bilden und das Zwischenstufenelement (11x) von einem Gelenkkern (11) gebildet ist, dessen Gelenkflächenmaße zwei unterschiedlichen Größenstufen angehören.

5. Satz von Bandscheiben-Endoprothesen bestehend aus mindestens zwei Größenstufen von Bandscheiben-Endoprothesen, die zwei mit je einem Wirbelkörper zu verbindende, über Gelenkflächen zusammenwirkende Abschlußplatten umfassen, deren Außenmaße sich von Größenstufe zu Größenstufe unterscheiden, **dadurch gekennzeichnet, daß** die Gelenkflächenmaße von Größenstufe zu Größenstufe übereinstimmen.

## Claims

1. A set of intervertebral disc endoprostheses comprising at least two size stages (a,b) of intervertebral disc endoprostheses, which comprise two end plates (1,2) to be connected to a respective vertebral body and co-operating via articulation surfaces, the outer and articulation surface dimensions of which vary from one size stage to another, **characterised in that** an intermediate stage element (1x,7x,11x) is provided forming an articulation surface (5), the articulation surface dimensions of which belong to a different size stage to its connecting surface dimensions on its side opposite the articulation surface.

2. A set of intervertebral disc endoprostheses according to Claim 1, **characterised in that** the intermediate stage element (1x) is an end plate.

3. A set of intervertebral disc endoprostheses according to Claim 1, **characterised in that** the intermediate stage element (7x) is a bearing element (7) retained via connecting surfaces (8) by an end plate (1,2), the connecting surface dimensions of which bearing element belong to a different size stage to its articulation surface dimensions.

4. A set of intervertebral disc endoprostheses according to Claim 1, **characterised in that** between each end plate (1,2) and an articulation core (11) the intervertebral disc endoprostheses each form a pair of articulation surfaces (10,12) and the intermediate stage element (11x) is formed by an articulation core (11) whose articulation surface dimensions belong to two different size stages.

5. A set of intervertebral disc endoprostheses comprising at least two size stages of intervertebral disc endoprostheses, which comprise two end plates to be connected with a respective vertebral body and co-operating via articulation surfaces, the outer dimensions of which vary from one size stage to another, **characterised in that** the articulation surface dimensions correspond from one size stage to another.

## Revendications

1. Jeu d'endoprothèses pour disques intervertébraux consistant en au moins deux catégories de dimensions (a, b) d'endoprothèses pour disques intervertébraux, qui comprennent deux plaques terminales (1, 2) à relier chacune à une vertèbre et coopérant par des surfaces d'articulation, dont les dimensions des surfaces externes et d'articulation diffèrent d'une catégorie de dimension à l'autre, **caractérisé par** le fait qu'un élément (1x, 7x, 11x) intermédiaire formant une surface d'articulation est prévu, dont les dimensions de surface d'articulation appartiennent à une catégorie de dimensions différente de celle de la surface de liaison sur sa face opposée à la surface d'articulation.

2. Jeu d'endoprothèses pour disques intervertébraux selon la revendication 1, **caractérisé par** le fait que l'élément intermédiaire (1x) est une plaque terminale.

3. Jeu d'endoprothèses pour disques intervertébraux selon la revendication 1 **caractérisé par** le fait que l'élément intermédiaire (7x) est un élément de palier (7) maintenu par une plaque terminale (1, 2) au moyen de surfaces de liaison (8) dont les dimensions de la surface de liaison appartiennent à une autre catégorie de dimension que ses dimensions de surface d'articulation.

4. Jeu d'endoprothèses pour disques intervertébraux selon la revendication 1, **caractérisé par** le fait que les endoprothèses pour disques intervertébraux forment entre chacune des plaques terminales (1, 2) et un moyen d'articulation (11) une paire de surfaces d'articulations (10, 12) et que l'élément intermédiaire (11x) est formé par un moyen d'articulation (11) dont les dimensions de surface d'articulation appartiennent à deux catégories de dimensions différentes.

5. Jeu d'endoprothèses pour disques intervertébraux constitué d'au moins deux catégories de dimensions d'endoprothèses pour disques intervertébraux, qui comprennent deux plaques terminales pour relier chacune à une vertèbre et coopérant au moyen de surfaces d'articulation, dont les dimensions extérieures se distinguent d'une catégorie de dimensions à l'autre, **caractérisé par** le fait que les dimensions des surfaces d'articulation correspondent d'une catégorie de dimensions à l'autre.
